# EUROPEAN PATENT APPLICATION

(11) **EP 4 656 200 A1**
(43) Date of publication of application: **03.12.2025**
(21) Application number: 25179312.1
(22) Date of filing: 28.05.2025
(51) Int. Cl.: A61K 38/08, A61P 19/00

(54) **PEPTIDE FOR USE IN SKELETON PROTECTION, AND IN INHIBITION OF PROLIFERATION AND MIGRATION OF SYNOVIOCYTES AND BONE RESORPTION**

(30) Priority: 31.05.2024 TW 113120387
(71) Applicant: Pell Bio-Med Technology Co., Ltd., Taipei City (TW)
(72) Inventor: LIN, Chen-Lung, Taipei City (TW); CHIEN, Wen-Pin, Taipei City (TW); WU, Hsin-Yi, Taipei City (TW); CHOU, Li-Che, Taipei City (TW); WU, Chiung-Ju, Taipei City (TW); YANG, Pei-Shan, Taipei City (TW)
(74) Representative: Cabinet Chaillot

(57) **Abstract**

Provided is a peptide for use in skeleton protection, comprising the amino acid sequence of SEQ ID NO: 1. The present invention proves that administering the peptide of SEQ ID NO: 1 can ameliorate cortical bone erosion of calcaneus bone resulting from rheumatoid arthritis (RA) and restore the decline of RA-induced bone mineral density. Therefore, the peptide of SEQ ID NO: 1 possesses a therapeutic effect for skeleton protection.

## Description

The present invention relates to a peptide for use in skeleton protection, comprising the amino acid sequence of SEQ ID NO: 1, especially a peptide for use in inhibiting bone resorption and the proliferation and migration of synoviocytes.

Rheumatoid arthritis (RA) is a multifactorial autoimmune disease that primarily manifests as chronic inflammatory arthropathy and is characterized by synovial hyperplasia which causes swelling, activated immune cells infiltration, synovium pannus invasion, and destruction of cartilage and bone, thereby resulting in deformity and disability.

Despite the pathogenesis of RA remains poorly understood, ample documents have demonstrated that fibroblast-like synoviocytes (FLSs) are the key factor of invasive synovium and play a critical role in the initiation and perpetuation of destructive joint inflammation. In healthy synovium, these cells mainly control the composition of the synovial fluid and maintain joint homeostasis. In RA, FLSs acquire an aggressive tumor-like phenotype characterized by resistance to apoptosis and increased migration with subsequent invasion to cartilage. FLSs also produce large quantities of inflammatory cytokines and chemokines, adhesion molecules, matrix metalloproteinases (MMPs), and tissue inhibitors of metalloproteinases (TIMPs), which are all involved in the progression of RA.

Bone erosion is a major outcome upon RA progression and correlates with disease severity and functional deterioration. It is thus usually seen in long-term RA patients that bone resorption (caused by osteoclasts) dominates over bone formation (caused by osteoblasts), leading to the destruction of subchondral bones. Recently, some articles demonstrated that FLSs are the principal factor responsible for differentiation of osteoclasts and bone erosion during inflammatory arthritis.

Conventional and biologic disease-modifying antirheumatic drug (DMARD) therapies have been proven to ameliorate the severity and progression of RA. However, a significant proportion of patients still do not achieve sustained remission. Humira (adalimumab, an anti-TNF-α antibody, for intravenous use) and Xeljanz (Tofacitinib, a JAK inhibitor; for oral use) are currently the two blockbuster drugs in clinical practice for treating RA. As the first JAK inhibitor, Tofacitinib was once the only available oral targeted drug for RA treatment. However, it incurred safety concerns associated with its mechanism of action, such as blood clot formation, abnormalities in liver enzymes, elevated cholesterol levels, and even severe infections and cancers.

Therefore, there is still an urgent need for a method for the treatment of symptoms resulting from RA.

In view of the deficit of the existing technology, the present invention provides a peptide for use in skeleton protection which can achieve the skeleton protection effect against the skeleton erosion resulting from rheumatoid arthritis.

To achieve the aforementioned purposes, the present invention provides a peptide, characterized for use in skeleton protection, comprising the amino acid sequence of SEQ ID NO: 1.

The present invention further provides a peptide for use in skeleton protection which consists of the amino acid sequence of SEQ ID NO: 1.

Preferably, the skeleton protection comprises ameliorating bone erosion.

Preferably, the skeleton protection comprises improving the surface smoothness of the cortical bone, thereby resulting in a smooth bone contour with integrity.

Preferably, the skeleton protection comprises improving or restoring bone mineral density (BMD).

According to the present invention, the skeleton comprises a cartilage, a bone, a ligament (connecting bones) and a joint. Therefore, the skeleton protection of the present invention comprises bone protection, cartilage protection and joint protection.

Preferably, the peptide is provided with a pharmaceutically acceptable carrier, comprising, but not limited to, a solvent, an emulsifier, a suspending agent, a decomposer, a binding agent, an excipient, a stabilizing agent, a diluent, a gelling agent, a lubricant, a surfactant or other similar or applicable carriers of the present invention.

Preferably, the peptide is provided in an enteral or parenteral dosage form. More preferably, the enteral dosage form comprises, but not limited to, an enteric-coated tablet, a multi-layered tablet, a sugar-coated tablet, a sublingual tablet, a chewable tablet, a buccal tablet, a capsule, a powder, a syrup, a solution, an emulsion, a suspension, a mucilage, a magmas, a fluidextract, an extract, a spirit, an elixir, a tincture, etc. or any other oral agent or an enema. More preferably, the parenteral dosage form comprises, but not limited to, an injection, an ointment, a lotion, a liniment, or an aerosolized spray.

The effective amount of the peptide for mice is 0.09 milligrams per kilogram per day (mg/kg/day) to 148 mg/kg/day. Preferably, the effective amount of the peptide for mice is 1.2 mg/kg/day to 125 mg/kg/day, or 0.1 mg/kg/day to 100 mg/kg/day. More preferably, the effective amount of the peptide for mice is 0.49 mg/kg/day to 99 mg/kg/day. More preferably, the effective amount of the peptide for mice is 0.5 mg/kg/day to 50 mg/kg/day. More preferably, the effective amount of the peptide for mice is 5 mg/kg/day to 30 mg/kg/day.

The effective amount of the peptide for humans is 0.007 mg/kg/day to 12 mg/kg/day. Preferably, the effective amount of the peptide for humans is 0.1 mg/kg/day to 10 mg/kg/day or 0.008 mg/kg/day to 8.13 mg/kg/day. More preferably, the effective amount of the peptide for humans is 0.04 mg/kg/day to 8 mg/kg/day. Further preferably, the effective amount of the peptide for humans is 0.04 mg/kg/day to 6.15 mg/kg/day. Such dose (effective amount of the peptide for humans) is obtained according to the method of "Estimating the maximum safe starting dose in initial clinical trials for therapeutics in adult healthy volunteers" published by the US Food and Drug Administration in 2005, which is determined based on an adult of 60 kg. For dose conversion, the suggested dose for humans per kilogram of body weight per day (/kg b.w./day) times 12.3 equals the dose for mice.

Preferably, the effective amount of the peptide for humans is 0.1 mg/kg/day to 10 mg/kg/day. More preferably, the effective amount of the peptide for humans is 0.5 mg/kg/day to 2 mg/kg/day.

Preferably, the peptide of the present invention is provided with a frequency of once a day to once a month. For example, once a day, twice a day, once a week, twice a week, or three times a week.

Preferably, the skeleton protection comprises avoiding cartilage damage.

Preferably, the avoiding cartilage damage is achieved by inhibiting the proliferation of synoviocytes.

Preferably, the avoiding cartilage damage is achieved by inhibiting the migration of synoviocytes.

Preferably, the synoviocytes are fibroblast-like synoviocytes. More preferably, the synoviocytes are rheumatoid arthritis fibroblast-like synoviocytes.

Preferably, the skeleton protection comprises joint protection.

Preferably, the skeleton protection comprises inhibiting bone resorption.

Preferably, the enteral dosage form is an oral dosage form.

Preferably, the parenteral dosage form is an injectable dosage form.

According to the present invention, the term "effective amount" refers to the dose required to demonstrate the desired biological response, which is the dose required to demonstrate a therapeutic effect. According to the present invention, the effective dose can achieve skeleton protection, amelioration of bone erosion, improvement of the surface smoothness of the cortical bone, improvement or restoration of bone mineral density, avoidance of invasion to cartilage, and inhibition of proliferation and migration of synoviocytes.

To achieve the aforementioned purposes, the present invention further provides a peptide for use in inhibiting the proliferation of synoviocytes, comprising the amino acid sequence of SEQ ID NO: 1.

To achieve the aforementioned purposes, the present invention further provides a peptide for use in inhibiting the migration of synoviocytes, comprising the amino acid sequence of SEQ ID NO: 1.

To achieve the aforementioned purposes, the present invention further provides a peptide for use in inhibiting bone resorption, comprising the amino acid sequence of SEQ ID NO: 1.

The present invention proves the peptide of SEQ ID NO: 1 has the advantages to ameliorate the cortical surfaces erosion of calcaneus bone in mice resulting from rheumatoid arthritis, and to restore the BMD destruction in mice resulting from rheumatoid arthritis, thereby achieving a skeleton protection effect. The peptide of SEQ ID NO: 1 can also inhibit the proliferation and migration of rheumatoid arthritis fibroblast-like synoviocytes, thereby avoiding joint and cartilage damage, and inhibit bone resorption to achieve a skeleton protection effect.
In the drawings:
FIG. 1A is the image of the mice calcaneus bone obtained firstly by micro-CT scanning and then segmented and analyzed by the CTAn software in Test 1.
FIG. 1B shows the magnified images of the vehicle group, the peptide of SEQ ID NO: 1 (10 mg/kg) treatment group and tofacitinib treatment group at Weeks 0, 4 and 6 selected from FIG. 1A.
FIGS. 2A to 2E show the bone mineral density of mice calcaneas bone of the vehicle group, tofacitinib treatment group and the peptide of SEQ ID NO: 1 (1 mg/kg, 3 mg/kg, and 10 mg/kg) treatment groups in Test 1, respectively. * indicates p<0.05, *** indicates p<0.001, and ns indicates no significant difference.
FIG. 3A is the cell proliferation rate of SW982 synoviocytes after the peptide of SEQ ID NO: 1 (at different concentrations) treatment in Test 2. ** means p<0.01 (compared with the vehicle group), *** means p<0.001 (compared with the vehicle group).
FIG. 3B is the cell proliferation rate of SW982 synoviocytes treated with IL-1β in Test 2.
FIG. 3C shows the inhibition effect on cell proliferation rate of SW982 synoviocytes after the co-treatment with the peptide of SEQ ID NO: 1 and IL-1β in Test 2.
FIG. 4A is the cell migration rate of SW982 synoviocytes after the peptide of SEQ ID NO: 1 (at different concentrations) treatment in Test 3. *** indicates p<0.001 (compared with the vehicle group).
FIG. 4B is the cell migration image of SW982 synoviocytes after the peptide of SEQ ID NO: 1 (at different concentrations) treatment for 24 hours in Test 3.
FIG. 5A shows the pit areas resulting from the coated calcium phosphate resorption in bone resorption assay.
FIG. 5B shows the inhibition effect on bone resorption of RAW264.7 cells after the co-treatment with the peptide of SEQ ID NO: 1 and RANKL in bone resorption assay.

The technical means adopted by the present invention to achieve the intended purpose are demonstrated with reference to the figures, the preparation examples and experimental examples shown below.

### Preparation example 1: the peptide of SEQ ID NO: 1

The peptide used in the present invention is the same as that described in Taiwan Patent Publication No. 1705818. Specifically, the standard Fmoc strategy and microwave peptide synthesizer were applied to synthesize the peptide of the present invention, which was Glp Glu Thr Ala Val Ser Ser His Glu Gln Asp, wherein the Glp was pyroglutamic acid (Cas Number 98-79-3). In general, Wang resin (0.6 mmol/g of load) preloaded with D residues was weighed into a reaction vessel and added with fresh dimethylformamide (DMF) (10 mL to 15 mL) before the synthesis, which was ready to be swollen. The swelling time was set as 3 minutes (min) on the microwave peptide synthesizer.

The first and subsequent Fmoc groups were removed by 5 mL 20% Piperidine in DMF by a standard de-protection process (as the "DEP" step) (in the first stage, the temperature was 75°C, the power was 155 W, and the temperature-holding time was 15 seconds; in the second stage, the temperature was 90°C, the power was 30 W, and the temperature-holding time was 50 seconds), in order to free the N-terminal amine group.

Then, 0.5 M N,N'-diisopropylcarbodiimide (DIC) in DMF was added to activate C terminus (as the "ACT" step), and the desired amino acid (as the Fmoc-AA (protected side chain)-OH) in a five-fold excess amount (with a concentration of 0.2 M in DMF) and 1.0 M Oxyma in DMF were added for coupling reaction (as the "AA" step). The standard coupling reaction was processed (in the first stage: the temperature was 75°C., the power was 170 W, and the temperature holding time was 15 seconds; in the second stage: the temperature was 90°C, the power was 30 W, and the temperature holding time was 230 seconds). After conducting "AA" for about 4 minutes, the Wang resin was washed with DMF solution. The steps of DEP-ACT-AA were repeated to construct a peptide from C-terminus to N-terminus.

Finally, the peptide was removed from the solid support by treating with 95% TFA/2.5% H₂O/2.5% TIPS in an ice bath, then allowing the reaction to come to room temperature for 2 hours. The filtrate was collected after filtration, iced ether was added for precipitation, and then centrifuged to remove supernatant. After that, ether was added to wash the precipitate. The aforementioned steps were repeated for 3 to 6 times. The final precipitate was taken out and freeze-dried to obtain a white floccus-like product, which is the final product. The purity of the final product was determined by high performance liquid chromatography, the molecular weight and the sequence were analyzed by LC-MS/MS, thereby obtaining the peptide of SEQ ID NO: 1.

### Test 1: Animal studies

This animal study was carried out according to the adjuvant-induced arthritis (AIA) model designed by Academia Sinica, Taipei, Taiwan. Specifically, arthritis induction was performed according to the method described in Gauldie, S.D., McQueen, D.S., Clarke, C.J. & Chessell, I.P. A, "Robust model of adjuvant-induced chronic unilateral arthritis in two mouse strains." J Neurosci Methods 139, 281-291 (2004). In short, this animal study comprised thirty C57BL/6 mice purchased from National Laboratory Animal Center, Taiwan, which were 8 weeks old with a body weight of about 20 to 25 grams. By using a 30-gauge needle mounted on a 100 µl Hamilton syringe, said mice were respectively injected with 5µg Complete Freund's Adjuvant (CFA) (F5881, Sigma, USA) in the right ankle joint once a week for consecutive 4 times, wherein the first injection was carried out at week 0 (day 0), and said mice were anesthetized and maintained with 2% isoflurane during the injection. The AIA model can induce long-term inflammation and swelling in unilateral anterior ankle joints in C57BL/6 mice, thereby simulating the clinical long-term chronic pain of patients with rheumatoid arthritis.

After CFA induction, said mice were weekly weighted and scored with rheumatoid arthritis score estimation (data not shown) according to Hsieh, W. S., et al. (2017). TDAG8, TRPV1, and ASIC3 involved in establishing hyperalgesic priming in experimental rheumatoid arthritis. Scientific reports,7(1), 8870. All mice showed sustained rheumatoid arthritis development at Week 4 (Day 28). The mice were assigned to 5 groups: (1) vehicle group, (2) P1 group (provided with 1 mg/kg/day peptide of SEQ ID NO: 1), (3) P3 group (provided with 3 mg/kg/day peptide of SEQ ID NO: 1), (4) P10 group (provided with 10 mg/kg/day peptide of SEQ ID NO: 1), and (5) T group (provided with 12.4 mg/kg/day tofacitinib). Said animal grouping was based on the rheumatoid arthritis scores by a random sampling mode. The drug administration from Week 4 (Day 28) to Week 6 (Day 42) was as follows: said mice received once-daily oral dosing (gavage) of 5 µL /g of body weight (bw) of a solution comprising: (1) the peptide of SEQ ID NO: 1 (1 mg/kg bw, 3 mg/kg bw or 10 mg/kg bw, dissolved in water) in P1, P3 and P10 groups, respectively, (2) tofacitinib (12.4 mg/kg bw, dissolved with 0.5% carboxymethylcellulose aqueous solution) in T group, and (3) water in vehicle group. Said mice were sacrificed on Day 45.

### A. Scanning by Micro-Computed Tomography (µCT)

Each mouse was anesthetized (2.5% isoflurane, 1.0 L/min air) and the anesthetic gas was maintained during the scanning. The mouse was subjected to lay on one side on the carbon fiber half-tube bed while kept anesthetized in the micro-CT scanner (Skyscan 1076), and the right hindpaw was positioned on a cylindrical styrofoam holder. The mouse's paw was immobilized at the scanning midline of the scanner to eliminate any undesirable movement of the paw.

Settings for the micro-CT scanner (SkyScan-1076; Bruker micro CT, Belgium) were as follows: isotropic voxel size: 9 µm, tube voltage: 49 kV, current: 167µA, Al filter: 0.5 mm, and 180° scan with 0.6° rotation step (720 projections) with frame averaging of 3. Subsequently, micro CT projections were back projection-reconstructed using the NRecon software (NReconServer64bit, Bruker, MicroCT, Belgium) and volume-rendered and visualized in 3D with the CTVox software (Bruker, MicroCT, Belgium). The images of calcaneus bone were segmented and analyzed using the CTAn software (Bruker, MicroCT, Belgium).

### B. Statistical Analysis

GraphPad Prism 9.3.1 (GraphPad Software, USA) was used for statistical analyses and graphing. Data are presented as mean ± standard deviation. The level of statistical significance was set at 5% (P< 0.05) for all analyses. Data analysis of the indexes derived from the µCT images of calcaneus bone of mice was carried out, for example, One-way RM ANOVA (One-way Repeated Measures Analysis of Variance) with Tukey's post hoc test was used for data analysis of bone mineral density.

### C. Preliminary safety profile

To assess the safety of the peptide of SEQ ID NO: 1, the aforementioned mice were weighed 6 times at one-week intervals and the body weight at Week 0 (before AIA induction) was used as the denominator to convert all data into the percentage of body weight changes. Both the peptide of SEQ ID NO: 1-treated and Tofacitinib-treated mice showed no significant difference in body weight changes after 1- or 2-week treatment (*i.e.,* Week 5 or Week 6) (P>0.05, compared with Week 4 or with the vehicle group) (data not shown). Observation of mice treated with the peptide of SEQ ID NO: 1 also showed no signs of irritability, loss of vigor, or any skin or hair changes. Therefore, the peptide of SEQ ID NO: 1 was recognized as safe for mice.

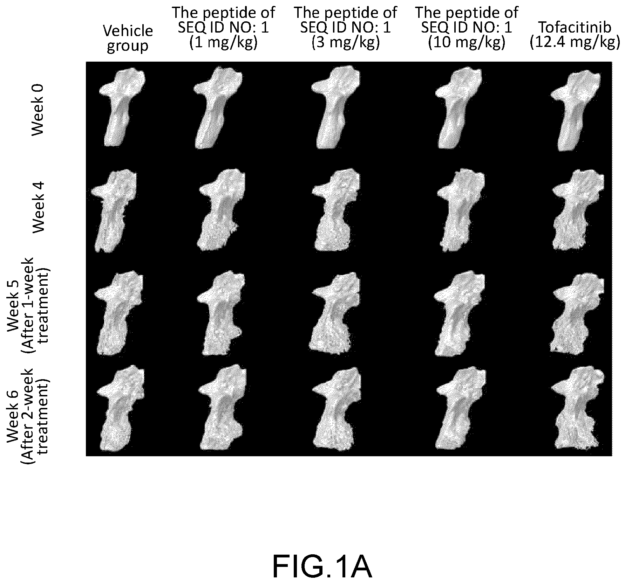

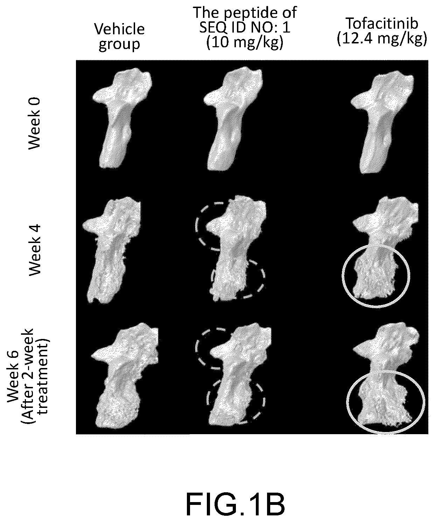

The observation time window of µCT scanning was set before AIA induction (Week 0), at the end of induction (Week 4), and after 1 or 2 weeks' treatment (*i.e.,* Week 5 and Week 6). The images were subsequently segmented and analyzed using the CTAn software, as shown in FIG. 1A. For clearer comparison, images at Weeks 0, 4 and 6 were magnified, as shown in FIG. 1B. According to FIG. 1A and FIG. 1B, obviously, the surface integrity of the calcaneus bone was disturbed at Week 4 (the end of induction and prior to treatment), manifested as unevenness of the cortical bone (dashed line circle, Week 4 vs Week 0). As shown in FIG. 1A, treatment of 1 mg/kg or 3 mg/kg of the peptide of SEQ ID NO: 1 shows no benefits in restoring the bone contour. In contrast, as shown in FIG. 1B, mice treated with 10 mg/kg of the peptide of SEQ ID NO: 1 for 2 weeks had much smoother cortical surfaces with fewer spiky projections of calcaneus bone (Week 6 vs Week 4, dashed line circle). It should be noted that Tofacitinib treatment did not reduce the extent of surface roughness or the distortion of the bone contour, and the bone destruction was even worse in some areas (circles in solid line, Week 6 vs Week 4). Therefore, 10 mg/kg of the peptide of SEQ ID NO: 1 can ameliorate bone destruction and demonstrated better therapeutic efficacy than the conventional drug of tofacitinib.

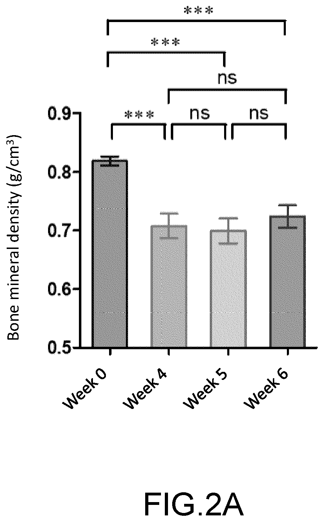

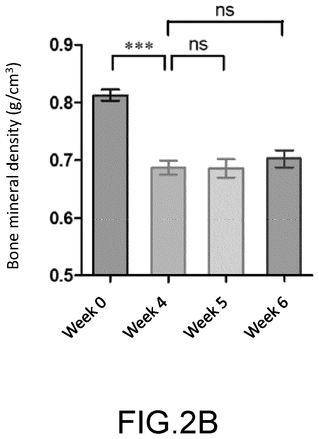

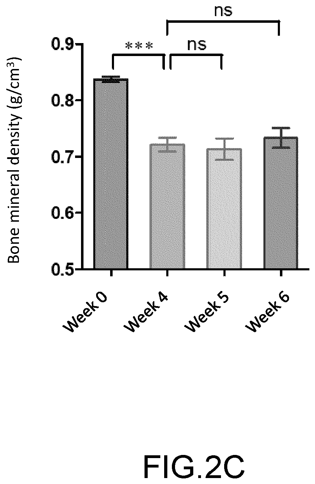

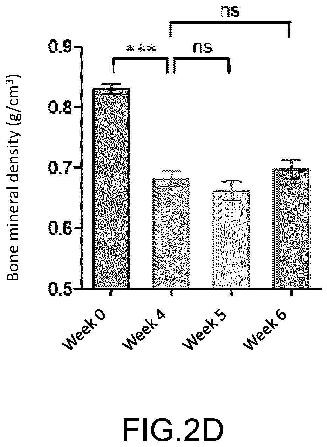

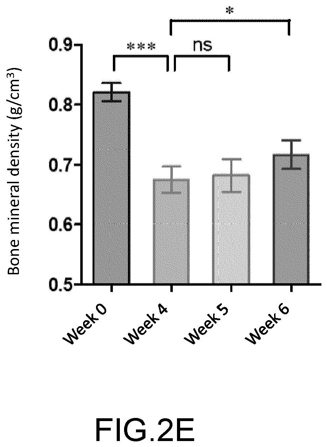

The determination result of the bone mineral density (BMD, in g/cm³) was shown in FIGS. 2A to 2E. The BMD was remarkably decreased at Week 4 as expected, and this phenomenon persisted to Week 6 (P<0.001, compared to Week 0; FIG. 2A). In addition, in T group, neither 1- nor 2-week treatment of Tofacitinib was able to revert the BMD decrement (P>0.05, Week 5/6 vs Week 4; FIG.2B). Further, in P1 and P3 groups, mice treated with 1 or 3 mg/kg of the peptide of SEQ ID NO: 1 had no significant changes in BMD after 1- or 2-week treatment (P>0.05, Week 5/6 vs Week 4; FIGS. 2C & D). In P10 group (provided with 10 mg/kg of the peptide of SEQ ID NO: 1), despite no increase in BMD after 1-week treatment, significant increases in BMD were observed after 2 week treatment (P<0.05, Week 6 vs Week 4, FIG. 2E), indicating that a relatively high dose (10 mg/kg) of the peptide of SEQ ID NO: 1 possesses a great therapeutic effect for bone protection in inflammatory arthritis. Therefore, 10 mg/kg of the peptide of SEQ ID NO: 1 can ameliorate bone destruction and restore the BMD of AIA mice, which demonstrated better therapeutic efficacy than that of tofacitinib.

### In vitro test

### Test 2: cell viability test

Owing to the restricted lifespan of primary rheumatoid arthritis fibroblast-like synoviocytes (RA-FLS), the fibroblast-like synoviocytes (FLS) cell line SW982 synoviocytes (hereinafter referred to as SW982 cells) are well accepted as a useful *in vitro* model for studying the pathogenesis of RA. In short, SW982 cells were seeded in a 96-well plate at a cell density of 6,000 cells per well. After the cells were treated in the following groups, the effect of each treatment group on the viability of SW982 cells was tested by means of the cell proliferation detection kit (cell counting kit-8, CCK8; Elabscience, Catalog No: E-CK-A361) according to the manufacturer's manual. Said treatment groups were as follows: (1) Different concentrations of the peptide of SEQ ID NO: 1: SW982 cells were treated with the L15 culture medium containing 10⁻⁶, 10⁻⁷, 10⁻⁸, 10⁻⁹ µM of the peptide of SEQ ID NO: 1 for 24 hours; (2) IL-1β stimulation group: SW982 cells were stimulated with the culture medium containing 1 ng/mL IL-1β for 24 hours to simulate the inflammatory synoviocytes of rheumatoid arthritis; (3) Co-treatment group: SW982 cells were treated with culture medium containing 10⁻⁹ µM of the peptide of SEQ ID NO: 1 and 1 ng/mL of IL-1β for 72 hours. The SW982 cells in said treatment groups were all cultured in a carbon dioxide-free environment. In addition, SW982 cells were simply cultured in L15 medium for 24 hours, as the control group. This test uses
2-(2-methoxy-4-nitrophenyl)-3-(4-nitrophenyl)-5-(2,4-disulfophenyl)-2H-tetrazolium sodium salt (WST-8) from CCK-8 kit to react with the dehydrogenase in viable cells, so that the color of all treatment groups containing WST-8 turned from pink to orange. By detecting the absorbance value at 450 nm, the activity of dehydrogenase was determined to calculate the number of viable cells.

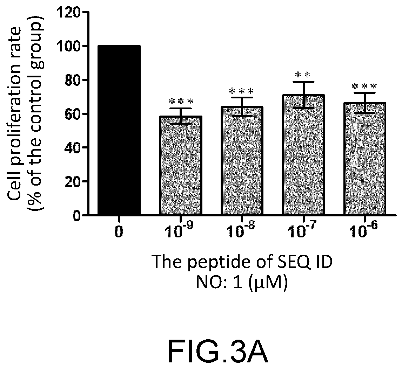

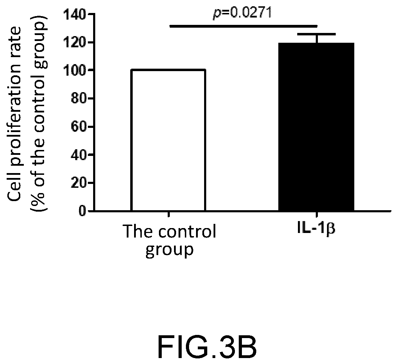

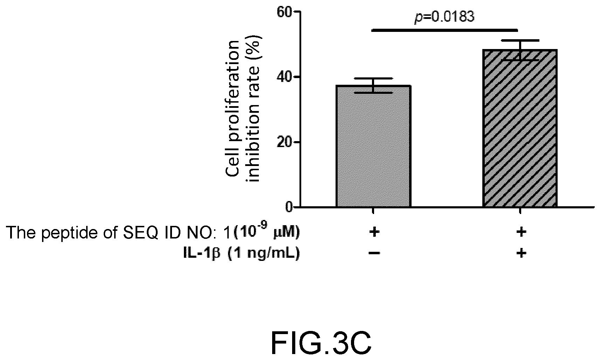

Results were shown in FIG. 3A, 3B and 3C. As shown in FIG. 3, the peptide of SEQ ID NO: 1 could effectively inhibit the proliferation of SW982 cells at concentrations ranging from 10⁻⁹ to 10⁻⁶ µM. As shown in FIG. 3B, treatment with IL-1β (1 ng/mL) for 24h dramatically enhanced the proliferation of SW982 cells, which was consistent with the fact that inflammatory factors in the microenvironment (as in RA) promote the proliferation of synovial fibroblasts. Of note, as shown in FIG. 3C, the peptide of SEQ ID NO: 1 (at 10⁻⁹ µM) can significantly suppress the IL-1β-induced proliferation of SW982 cells.

### Test 3: SW982 cell migration measurement

It is known that fibroblast-like synoviocytes secrete inflammatory cytokines, chemokines, and matrix metalloproteinases (MMPs) and induce cell migration and invasion to damage joints and cartilage. We next examined the effect of the peptide of SEQ ID NO: 1 on SW982 cell migration through a conventional wound healing experiment. Specifically, a Culture-Insert 2 Well (ibidi) in which two cell culture reservoirs were separated by a 500 µm wall was placed on a cell culture plate surface. Both reservoirs were filled with cells suspension containing L15 culture medium and 4x10⁴ SW982 cells to allow cell growth in the designated areas. After appropriate cell attachment of all SW982 cells, the Culture-Insert 2 Well was removed to create a cell-free gap of approximately 500 µm. The cell monolayer was washed with PBS buffer to remove detached cells and debris. The SW982 cells were incubated with the peptide of SEQ ID NO: 1 (10⁻⁵, 10⁻⁶, 10⁻⁷ µM) in L15 culture medium or with L15 culture medium only (as control group) for 24h in a carbon dioxide-free environment. By using direct microscopic visualization, a reference point at the bottom was created in each field, and the image of the remaining cell-free area in the identical field shot immediately after 24h-treatment of the peptide of SEQ ID NO: 1 was measured to analyze cell migration and wound healing rates. Image J (National Institutes of Health, NIH) software was used to analyze the wound healing/cell migratory dynamics. Specifically, Image J was used to calculate the area of the cell-free area, and the calculation formula of migration rate is as follows: (Size of cell-free area before peptide treatment - Size of cell-free area after peptide treatment) ÷ Size of cell-free area before peptide treatment × 100%.

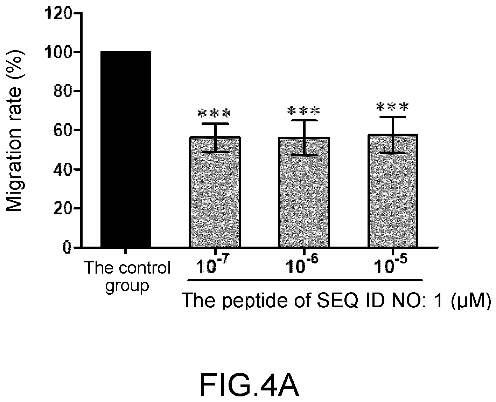

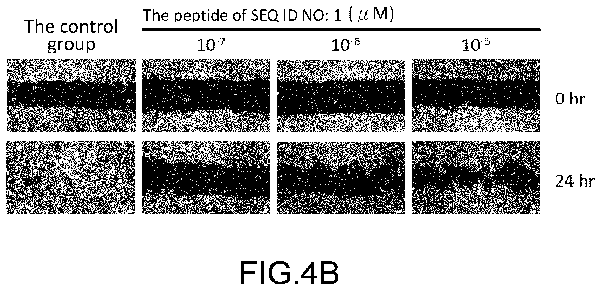

Results were shown in FIGs. 4A and 4B. As shown in FIG. 4A, 24h-treatment of the peptide of SEQ ID NO: 1 (10⁻⁷ to 10⁻⁵ µM) significantly reduced the lateral migration of SW982 cells. The cell images were shown in FIG. 4B. It should be noted that despite a lower concentration of the peptide of SEQ ID NO: 1 (10⁻⁵ µM) might exert stronger inhibition than a higher concentration (10⁻⁷ µM) as shown in FIG. 4B, no statistical significance was found in the results of FIG. 4A.

From above, the peptide of SEQ ID NO: 1 was confirmed by the present invention to actually ameliorate the cortical surface erosion of calcaneus bone in mice resulting from RA and restore BMD destruction in mice resulting from RA, thereby achieving a skeleton protection effect. The peptide of SEQ ID NO: 1 can also inhibit the proliferation and migration of SW982 synoviocytes, thereby avoiding joint and cartilage damage.

### Test 4: Bone resorption assay

This test used RANKL (receptor activator of nuclear factor kappa-B ligand) as an inducer of osteoclastic differentiation to detect whether the peptide of SEQ ID NO: 1 could inhibit osteoclast resorptive function induced by RANKL *in vitro,* and comprised three groups: control group, RANKL group and treatment group.

First, RAW264.7 cells were seeded in a calcium phosphate (CaP)-coated 96-well with a density of 2 × 10³ cells per well. RAW264.7 is a macrophage cell line, and RAW264.7 cells can differentiate into an osteoclast. The cells of all groups were cultivated for 7 days according to the manufacturer's instructions of Bone Resorption assay kit (Cosmo Bio). The differences between control group, RANKL group and treatment group were as follows:
(1) Control group: neither RANKL nor the peptide of SEQ ID NO: 1 was provided to RAW264.7 cells during a 7-day incubation period.
(2) RANKL group: RANKL in a concentration of 100 ng/ml was provided to RAW264.7 cells at Day 1 and incubated for 7 days.
(3) Treatment group: RANKL in a concentration of 100 ng/ml and the peptide of SEQ ID NO: 1 in a concentration of 100 µM were simultaneously provided to RAW264.7 cells at Day 1 and incubated for 7 days.

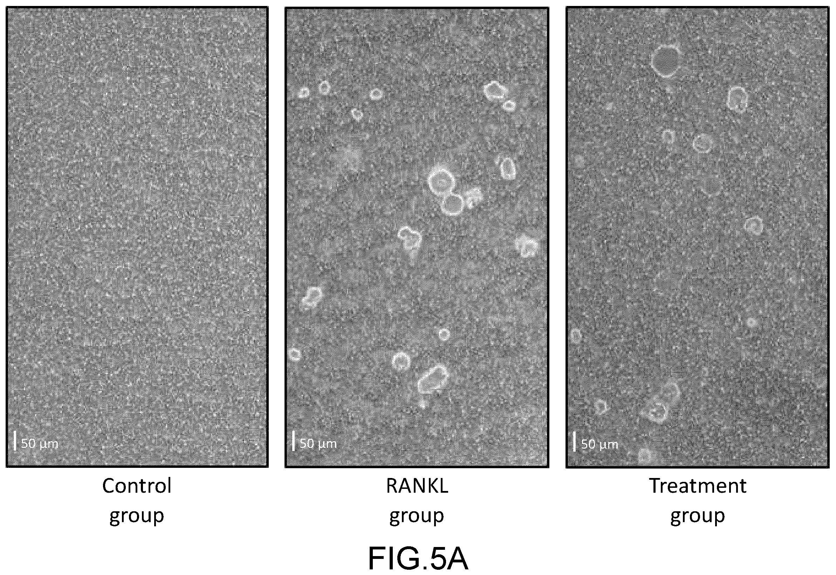

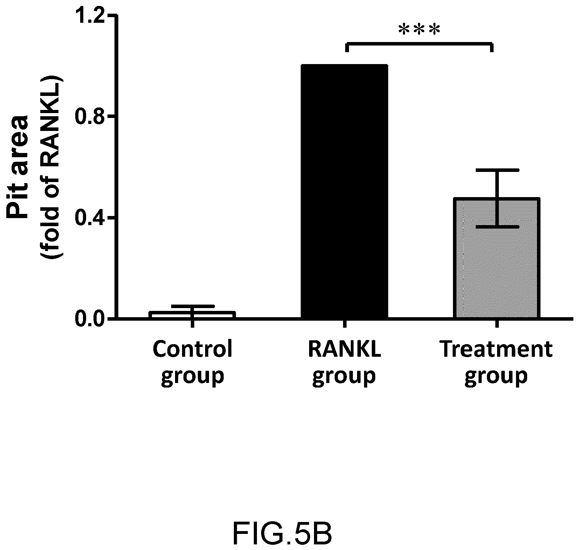

After the 7-day incubation period, all wells of all groups were washed with 0.2 ml to 0.5 ml of 5% sodium hypochlorite to remove the cells adhered to the surface. The plates of all groups were further washed with water and dried. The regions in each well of all groups were photographed by a light microscope with a magnification of 100 folds and as shown in FIG. 5A, wherein the pit areas thereon resulting from the coated calcium phosphate resorption by osteoclasts were measured and calculated by image analyzing software (Image J). The result of pit area representing the mean ± SD of three independent samples was shown in FIG. 5B, wherein *** indicated p <0.001 relative to the RANKL group.

According to FIG.5B, the pit area of the RANKL group was recognized as one-fold, which was significantly higher than that of the treatment group (about 0.5 fold). Thus, it is found that the peptide of SEQ ID NO: 1 can also inhibit the calcium phosphate resorption by osteoclasts (bone resorption), thereby avoiding joint and cartilage damage.

The above embodiments are only preferred embodiments of the present invention, not intended to limit the present invention in any aspect. Although the present invention has been described in terms of specific preferred embodiments, it should be understood that the invention should not be unduly limited to those specific embodiments. It is apparent to those skilled in the art that various modifications and variations can be made without departing from the scope and spirit of the invention. In fact, the various modifications that are obvious to those of ordinary skill in the art are also encompassed within the scope of the following claims.

## Claims

1. A peptide for use in skeleton protection, **characterized in that** the peptide comprises the amino acid sequence of SEQ ID NO: 1.

2. The peptide as claimed in Claim 1, wherein the skeleton protection comprises ameliorating bone erosion.

3. The peptide as claimed in Claim 1, wherein the skeleton protection comprises improving the surface smoothness of the cortical bone.

4. The peptide as claimed in Claim 1, wherein the skeleton protection comprises improving or restoring bone mineral density.

5. The peptide as claimed in any one of Claims 1 to 4, wherein an effective amount of the peptide is 0.1 mg/kg/day to 10 mg/kg/day.

6. The peptide as claimed in Claim 1, wherein the skeleton protection comprises avoiding cartilage damage.

7. The peptide as claimed in Claim 6, wherein the avoiding cartilage damage is achieved by inhibiting the proliferation of synoviocytes.

8. The peptide as claimed in Claim 6, wherein the avoiding cartilage damage is achieved by inhibiting the migration of synoviocytes.

9. The peptide as claimed in Claim 7 or 8, wherein the synoviocytes are fibroblast-like synoviocytes.

10. The peptide as claimed in Claim 1, wherein the skeleton protection comprises inhibiting bone resorption.

11. The peptide as claimed in Claim 1, wherein the skeleton protection comprises joint protection.

12. A peptide for use in inhibiting proliferation of synoviocytes, **characterized in that** the peptide comprises the amino acid sequence of SEQ ID NO: 1.

13. A peptide for use in inhibiting migration of synoviocytes, **characterized in that** the peptide comprises the amino acid sequence of SEQ ID NO: 1.

14. A peptide for use in inhibiting bone resorption, **characterized in that** the peptide comprises the amino acid sequence of SEQ ID NO: 1.
